(19)

**Europäisches Patentamt**
**European Patent Office**
**Office européen des brevets**

(11) **EP 3 359 156 B1**

(12) **EUROPEAN PATENT SPECIFICATION**

(45) Date of publication and mention
of the grant of the patent:
**20.07.2022   Bulletin 2022/29**

(21) Application number: **16853180.4**

(22) Date of filing: **07.10.2016**

(51) International Patent Classification (IPC):
**A61K 31/4985** (2006.01)    **A61K 31/40** (2006.01)
**A61K 31/506** (2006.01)    **A61K 9/20** (2006.01)
**A61K 31/515** (2006.01)    **A61K 31/522** (2006.01)
**A61K 9/46** (2006.01)    **A61K 31/403** (2006.01)

(52) Cooperative Patent Classification (CPC):
**A61K 9/0007; A61K 9/2018; A61K 31/40;**
**A61K 31/403; A61K 31/4985; A61K 31/515;**
**A61K 31/522**

(86) International application number:
**PCT/IB2016/056010**

(87) International publication number:
**WO 2017/060861 (13.04.2017 Gazette 2017/15)**

(54) **RAPIDLY DISINTEGRATING TABLET COMPOSITIONS OF DPP-IV INHIBITORS WITH LOW MINERAL CONTENT**

SCHNELL ZERFALLENDE TABLETTENZUSAMMENSETZUNGEN VON DPP-IV-HEMMERN MIT NIEDRIGEM MINERALGEHALT

COMPOSITIONS DE COMPRIMÉS À DÉLITATION RAPIDE D'INHIBITEURS DE DPP-IV À FAIBLE TENEUR EN MINÉRAUX

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority:  **07.10.2015   IN 5379CH2015**

(43) Date of publication of application:
**15.08.2018   Bulletin 2018/33**

(73) Proprietor: **Steerlife India Private Limited**
**Bengaluru, Karnataka 560058 (IN)**

(72) Inventors:
• **PADMANABHAN, Babu**
**Bengaluru 560003 (IN)**
• **SEN, Himadri**
**Pune**
**Maharashtra (IN)**
• **BHUSHAN, Indu**
**Bengaluru**
**Karnataka 560058 (IN)**

• **KULKARNI, Vijay**
**Bengaluru**
**Karnataka 560058 (IN)**
• **SHETTY, Rakshith**
**Bengaluru**
**Karnataka 560058 (IN)**
• **GURRAM, Aravind Kumar**
**Bengaluru**
**Karnataka 560058 (IN)**

(74) Representative: **Müller Schupfner & Partner**
**Patent- und Rechtsanwaltspartnerschaft mbB**
**Schellerdamm 19**
**21079 Hamburg (DE)**

(56) References cited:
**EP-A1- 2 848 241**    **WO-A1-95/34293**
**WO-A1-2015/040460**    **WO-A1-2015/044880**
**WO-A1-2017/098481**    **WO-A2-2011/012322**
**US-A1- 2014 287 040**

**Description**

**FIELD OF INVENTION**

The present disclosure relates to rapidly disintegrating tablet compositions of DPP-IV inhibitors for the treatment of diabetes having mineral content below the daily intake limits. The present disclosure also relates to a simple and convenient continuous process for the preparation of the rapidly disintegrating tablet compositions of DPP-IV inhibitors. These compositions are particularly suitable for treatment of patients having diabetes with comorbid cardiovascular disease or those having diabetes and are at a risk of cardiovascular disease.

**BACKGROUND OF THE INVENTION**

As per WHO factsheet, about 347 million people worldwide have diabetes. Type 2 diabetes accounts for around 90% of all diabetes worldwide. Diabetes has been associated with an increase in the risk of cardiovascular disease (CVD), cardiovascular death and all-cause mortality and CVD risks are even higher in those with concurrent hypertension. About 20-60% of people with Type 2 diabetes have hypertension. Majority of people with diabetes die due to cardiovascular causes. Mineral levels assume great significance in Type 2 diabetes patients with such cardiovascular complications. For example, excess sodium intake in these cases contributes to hypertension both directly, by increasing intravascular volume, and indirectly, by blunting the effectiveness of antihypertensives. Excess sodium intake is also associated with worsening of proteinuria in patients with microalbuminuria and decreases the antiproteinuric effects of antihypertensives. Consequently, a sodium-restricted diet has long been a first line of intervention for people with hypertension and is particularly important in those with Type 2 diabetes. Similarly, potassium is a vital electrolyte. Both high and low levels are associated with various medical conditions, including hypertension, cardiac arrhythmias, osteoporosis and nephrolithiasis. Low potassium levels can cause muscle weakness and heart rhythm disturbances in patients with Type 2 diabetes having a comorbid cardiovascular disease or those who are at risk of developing a cardiovascular disease. Excess potassium can cause dangerous heartbeat irregularities and even sudden death in such patients. Apart from this, medications can also cause fluctuations in potassium levels in the blood. For example, diuretics administered to treat heart failure and high blood pressure cause low potassium levels in the blood. Insulin administered to diabetic patients decreases potassium levels in the blood by redistributing it into cells and a large part of the shifted extracellular potassium is excreted in urine because of osmotic diuresis leading to hypokalemia. Hypokalemia increases the risk of dangerous irregularities in the heart rate.

The enzyme dipeptidyl peptidase IV (DPP-IV) is a key regulator of insulin-stimulating hormones, glucagon-like peptide (GLP-1) and glucose-dependent insulinotropic polypeptide (GIP), and is a promising target for the treatment of Type 2 diabetes. DPP-TV inhibitors are used for the treatment of Type 2 diabetes. Drug regulatory authorities such as the USFDA, have approved DPP-IV inhibitors for use as monotherapy in Type 2 diabetes. DPP-IV inhibitors can also be administered in combination to patients already on metformin, sulfonylureas, thiazolidinediones or insulin. Sitagliptin, Saxagliptin, Linagliptin, Alogliptin, Vildagliptin and Teneligliptin are some of the examples of DPP-IV inhibitors currently approved for the treatment of Type 2 diabetes.

Rapidly disintegrating tablets (RDT) that dissolve or disintegrate rapidly in oral cavity, are a desirable alternative for administration of DPP-IV inhibitors to geriatric patients having difficulty in swallowing tablets. In addition to improved palatability they also provide advantages such as patient compliance, quick onset of action, improved bioavailability, etc.

In order to be effective, RDTs need to disintegrate rapidly and at the same time, need to have adequate mechanical strength to withstand the rigors of handling and transportation. Many strategies have been tried in the past, for achieving high porosity (for rapid disintegration) and adequate tablet strength in RDTs. Several granulation methods like wet granulation, dry granulation, spray drying, and flash heating have been tried to obtain granules suitable for making RDTs with less than satisfactory results. Another approach was to select special types of excipients to formulate the RDTs. Yet another approach was, to compress tablets at low pressure and apply various treatments to the soft tablets. But these methods were not satisfactory to provide for a low-cost economic and simple alternative to make fast disintegrating tablets. For example, in preparation of RDTs using dry granulation, excipients like higher density alkali earth metal salts and water-soluble carbohydrates do not provide quick disintegration and a smooth mouth feel. Low-density alkali earth metal salts and water soluble carbohydrates are also difficult to compress and cause inadequate content uniformity. For these reasons, low-density alkali earth metal salts or water-soluble carbohydrates need to be pre-compacted, before being compressed into tablets.

Mechanical strength and good disintegration of the tablets were improved by using non conventional equipment and/or multistage processes. Using a non-conventional approach, however, required substantially larger investment in machinery. For example, Zydis® technology based on freeze drying was popularly used to prepare fast disintegrating tablets. However, the drug was required to be chemically stable and water insoluble, with a particle size smaller than 50 μm. The units were fragile and light-weight, and could not be packed in a conventional blister. A special peelable backing foil was needed to package the Zydis® units. Also the Zydis® formulation had a high sensitivity to moisture. In Lyoc® formulations, porous solid forms were obtained by freeze drying an oil-in-water emulsion placed directly in blister pockets. In order to prevent inhomogeneity by sedimentation during freeze drying, these formulations required a large proportion of undissolved inert filler to increase the viscosity of the suspension. The high proportion of filler caused reduction in porosity of the tablet, and resulted in slower disintegration. The units also had poor mechanical resistance. Similarly, molded tablets also did not have mechanical strength. If hardness-enhancing agents were added to the formulation of molded tablets, the rate of tablet disintegration usually decreased. OraSolv® technology produced tablets by low compression pressure. It used effervescent excipients that released gas upon contact with water. Because of the soft and fragile nature of OraSolv® tablets, a special packaging system, known as PakSolv®, was developed to protect the tablets from breaking during transport and storage. Melt granulation fast disintegrating tablets had better hardness results than the wet granulation fast disintegrating tablets but disintegration time of melt granulation tablets, was more than a minute.

WO2015040460A1 discloses effervescent compositions of antidiabetic compounds free from sodium introduced by the effervescing couple. However, such compositions comprise high potassium content. Therefore, there is a long felt need in the art to provide RDT compositions of DPP-IV inhibitors which disintegrate rapidly and yet have adequate mechanical strength and also have low mineral content.

WO 2015/044880 A1 discloses an oral liquid composition comprising sitagliptin phosphate, xylitol, potassium bicarbonate, hydrochloric acid, sucralose, strawberry flavor, purified water but does not disclose compositions having a sodium content of 1 to 10 mg and a potassium content which is less than 15 mg in one tablet.

EP 2848241 A1 discloses an effervescent formulation comprising the following components: a. 2.0 - 80.0% by weight of linagliptin, b. 0.1 - 15.0% by weight of polyethylene glycol, c. 2.0 - 90.0 %-by weight of sodium glycine carbonate, d. 2.0-90.0% by weight of fumaric acid, e. 5.0 - 90.0 % by weight of lactose, f. 0.1 - 0.2 % by weight of sweetener, g. 0.1 - 5.0 % by weight of aromatic agent. The disclosed formulation has got a sodium content above 10 mg and a potassium content which is above 15 mg in one tablet.

## SUMMARY OF THE INVENTION

The present disclosure provides a rapidly disintegrating tablet comprising a therapeutically effective amount of a DPP-IV inhibitor, and an acid-base couple. The present disclosure provides a rapidly disintegrating tablet comprising a therapeutically effective amount of a DPP-IV inhibitor, and an acid-base couple wherein the tablet has a sodium content of 1 mg to less than 10 mg and a potassium content less than 15 mg.

The present disclosure also provides a process for preparation of a rapidly disintegrating tablet comprising a DPP-IV inhibitor, the process comprising feeding an input blend comprising an acid-base couple, and optionally a DPP-IV inhibitor, into a co-rotating twin screw processor, melting only a portion of the acid to serve as an *in situ* granulating agent, granulating the input blend to form granules, collecting granules from the co-rotating twin screw processor, mixing the granules with one or more excipients and optionally, with a DPP-IV inhibitor to obtain a blend and compressing the blend to form one or more tablets - according to claim 9. . Herein, the DPP-IV inhibitor is either added to the input blend or mixed with the granules.

The present disclosure also provides a rapidly disintegrating tablet comprising a therapeutically effective amount of a DPP-IV inhibitor, and an acid-base couple, wherein the disintegration time of the tablet does not exceed 120 seconds for upto 50% loss of porosity.

The present disclosure also provides a rapidly disintegrating tablet comprising a therapeutically effective amount of a DPP-IV inhibitor, and an acid-base couple, wherein the disintegration time of the tablet does not exceed 120 seconds for loss of porosity between 30-50% (according to claim 11).

The present disclosure also provides a rapidly disintegrating tablet comprising a therapeutically effective amount of a DPP-IV inhibitor, and an acid-base couple, wherein the disintegration time of the tablet does not exceed 60 seconds for loss of porosity between 30-45 % (according to claim 12).

## DETAILED DESCRIPTION OF THE INVENTION

The present disclosure relates to rapidly disintegrating tablet (RDT) compositions comprising DPP-IV inhibitors. The RDT compositions of the present disclosure comprise at least one acid-base couple, have low content of sodium and potassium and are therefore, suitable for administration to diabetic patients. The content of sodium and/or potassium in the compositions is below the daily intake limits of sodium and potassium recommended in case of Type 2 diabetes patients. The RDT compositions are rapidly disintegrating and can be administered as effervescent, dispersible, soluble or orally disintegrating tablets.

In accordance with an embodiment, the RDT composition comprises an acid component comprising a combination of a portion of the acid that has been melted and a portion of the acid that has not been melted and, a base component comprising a carbonate functional group, wherein the base component is capable of reacting with the acid component to form carbon dioxide and wherein the composition does not contain an affirmatively added granulating agent. The acid-base couple in the present disclosure is preferably used in an amount suitable to cause a disintegration of the tablets while avoiding the generation of fizz which is characteristic of a typical effervescent composition.

The term "DPP-IV inhibitors" as used herein includes active ingredients that inhibit the degradation of incretins such as GLP-1 by inhibiting the enzyme dipeptidyl peptidase IV (DPP-IV). It also includes their pharmaceutically acceptable salts, esters or derivatives. The hydrate or anhydrous forms or combinations thereof of the DPP-IV inhibitors are also included in the said definition. DPP-IV inhibitors include those which, when administered individually to a patient having hyperglycemia, cause 10-35 mg/dl reduction in fasting plasma glucose. DPP-IV inhibitors also include those which, when administered individually to a patient having hyperglycemia, cause 20-60 mg/dl reduction in post-prandial glucose or 0.4 to 1.2% reduction in HbA1c. DPPIV inhibitors can be selected from a group consisting of but not limited to Sitagliptin, Saxagliptin, Linagliptin, Alogliptin, Vildagliptin, Gemigliptin and Teneligliptin, their pharmaceutically acceptable salts and combination thereof. The therapeutically effective amount for a DPPIV inhibitor is the dose which, when administered individually to a patient having hyperglycemia, causes 10-35 mg/dl reduction in fasting plasma glucose or 20-60 mg/dl reduction in post-prandial glucose or 0.4 to 1.2% reduction in HbA1c. The amount of the DPP-IV inhibitor may be the therapeutically effective amount of the DPP-IV inhibitor and varies as per potency for each DPP-IV inhibitor. Such therapeutically effective amounts can also be derived from the database of labels of the approved drug products as published by the USFDA. For example, the therapeutically effective amount of Sitagliptin may range from about 1-100 mg and of Saxagliptin may range from about 2-5 mg.

The terms "rapidly disintegrating tablets" or "RDTs" refer to tablets that can be orally disintegrating, effervescent or dispersible. Orally disintegrating tablets are those which disintegrate rapidly within seconds when placed in the oral cavity. Effervescent tablets are those which contain acid substances and carbonates or hydrogen carbonates that react rapidly in presence of water to release carbon dioxide (characterized typically by generation of a fizz) and are intended to be dissolved in water before administration. Dispersible tablets are those which are intended to be dispersed in water before administration giving a homogeneous dispersion.

The term "Percentage Loss of porosity" refers to the loss in porosity of tablet blend (which is ready for compression) when such a blend is compressed to form a tablet. Calculation of % loss of porosity is elaborately explained in the examples.

The RDTs of the present disclosure essentially comprise an acid-base couple. In accordance with an embodiment, the acid can be selected from one or more of organic acids present as a free acid, acid anhydride or its salt form. Specifically, the acid can be selected from citric acid, tartaric acid, fumaric acid, malic acid, adipic acid, succinic acid, lactic acid, glycolic acid, alpha hydroxy acid, ascorbic acid, amino acid or its alkali hydrogen acid salts. For example, in some embodiments, the acid is citric acid.

In accordance with an embodiment of the present disclosure, the base is a carbon dioxide precursor and can be selected from one or more of carbonate-containing compounds, such as carbonate, sesquicarbonate or bicarbonate salts of potassium, lithium, sodium, calcium, or ammonium; L-lysine carbonate; arginine carbonate; sodium glycine carbonate; or sodium amino acid carbonate. Mixtures of one or more bases can also be used. For example, in some embodiments, the base is a mixture of sodium carbonate and sodium bicarbonate or a mixture of sodium carbonate and potassium bicarbonate or a mixture of potassium bicarbonate and potassium carbonate or a mixture of potassium carbonate and sodium bicarbonate.

In accordance with an exemplary embodiment, an anhydrous form of the acid is used. In accordance with an exemplary embodiment, an anhydrous form of the base is used. In yet another embodiment, both the anhydrous acid and the anhydrous base can be used.

The amount of the acid and the base in the RDT compositions can vary depending on the acid and the base used. The acid and base can be present in a molar ratio ranging from 3:1 to 1:6, preferably from 1:2.5 to 1:3.5. In exemplary embodiments, the weight ratio of the acid and the base can be selected from 1:1.1 to 1:1.53.

In accordance with an embodiment of the present disclosure, the RDT composition comprises a DPP-IV inhibitor and an acid-base couple and the composition has a sodium content less than 10 mg and a potassium content less than 15 mg. In accordance with an aspect, the said RDT composition has a sodium content in the range of about 1-10 mg and a potassium content less than 15 mg.

The present disclosure provides a robust method to prepare RDTs by a simple and convenient formulation process. In an embodiment, the present disclosure provides a continuous process for preparation of granules that can be used to prepare the RDT compositions comprising DPP-IV inhibitors. In an embodiment, the present disclosure provides a twin screw granulation process for the continuous preparation of granules that can be used to prepare the RDT compositions comprising DPP-IV inhibitors. Such a granulation process is carried out in a twin screw processor preferably in a co-rotating twin screw processor.

The co-rotating twin-screw processor has two co-rotating screws inside a processor barrel. These screws are open length wise, and closed cross wise. Besides being intermeshing and self wiping, radial forces are found to be uniformly distributed, thus enabling them to be operated at a high screw speed. The twin screw processor has a modular design for barrels and screws. Segmented screws convey and shear the materials in channels bounded by screw flights and barrel walls, with short mass transfer distances. Each individual screw section can be designed to perform specific functions such as; conveying, mixing, shearing, or pressure building, thus allowing precise control of conditions along the screw length. The screw elements differ in pitch, pitch direction, length, and angle of offset. Pitch, length, and location of such screw elements on the shaft define a screw profile that influences the product characteristics. Due to variable screw configuration, twin screw processors provide greater flexibility of operations to control characteristics of product by monitoring and regulating residence time, product temperature, pressure, and shear. The processing aspects and parameters related to processing of granulating blend in a twin screw processor are elaborately explained in the examples. Also, the co-pending Indian patent application 4527/CHENP/2014 discloses methods and systems for processing granulation blend in a twin screw processor

In a further embodiment, the present disclosure relates to a process for preparation of an RDT composition comprising a DPP-IV inhibitor and an acid-base couple comprising the step of processing an input blend comprising the acid-base couple and, optionally a DPP-IV inhibitor, through a twin screw processor. Such an RDT composition can suitably contain sodium and potassium in an amount less than their daily recommended intake for diabetic patients, more specifically less than the daily intake recommended for diabetic patients with co-morbid cardiovascular disorder.

Thus, in accordance with a yet further embodiment, the present disclosure relates to an RDT composition comprising a DPP-IV inhibitor, and an acid-base couple, wherein the composition has a sodium content in the range of about 1- 10 mg and a potassium content less than 15 mg wherein, the RDT composition is prepared using a twin screw processor.

Granules comprising the acid-base couple can be prepared using the twin screw processor and the DPP IV inhibitor can subsequently be mixed homogeneously with the granules i.e. extragranularly or alternatively, the DPP-IV inhibitor can be processed in the twin screw processor along with the acid-base couple to incorporate it within the granules i.e. intragranularly. Depending on the dosage form, one or more excipients can be included in the RDT compositions comprising DPP-IV inhibitor intragranularly or extragranularly. Suitable excipients are selected from a group consisting of fillers, disintegrants, surfactants, taste modifiers, soluble and insoluble lubricants, flavoring substances, coloring agents, sweeteners or combinations thereof.

Suitable fillers include but starch, mannitol, sorbitol, lactose, microcrystalline cellulose and combinations or co-agglomerates thereof. In an exemplary embodiment, PEARLITOL® Flash which is a co-agglomerate of starch and mannitol is used. The amount of the filler in the composition can be from about 25-70% w/w, preferably about 25-68% w/w of the composition.

Suitable disintegrants include natural, modified or pregelatinized starch, crospovidone, croscarmellose sodium, sodium starch glycolate, low-substituted hydroxypropyl cellulose, crospovidone, calcium silicate and starch or mixtures thereof. The amount of the disintegrant in the composition can be from about 2-30% w/w of the composition.

Suitable surfactants include, sodium docusate, polyoxyethylene sorbitan fatty acid esters, sodium lauryl sulfate, sorbic acid, sorbitan fatty acid esters, and mixtures thereof.

Suitable taste modifiers include ion exchange resins which are either strong or weak cation or anion exchangers which form a resin complex with the drug (such as commercially available Amberlite® IRP 69 or 88, Tulsion® T-335, T-339, Indion® 204,214 etc.), oils, surfactants, polyalcohols, lipids, lecithins, salts of metals (such as sodium chloride, sodium acetate, sodium gluconate), amino acids such as glycine, taurine, alanine, adsorbents (such as magnesium aluminum silicate, zeolite®, silica, clay). The compositions may also include salivating agents including, but not limited to, micronized polyethylene glycol, sodium chloride or precipitated micronized silica.

Suitable sweeteners include acesulfame potassium, aspartame, saccharin, sucralose, neotame, advantame, sucrose and combinations thereof. The amount of the sweetener in the composition can be from about 0.25-1% w/w of the composition.

Suitable soluble lubricants include polyethylene glycols (PEG), for example- PEG 4000, PEG 6000 and PEG 8000, polyoxyethylene stearate and sodium or magnesium lauryl sulphate, sodium benzoate, potassium sorbate, L-leucine and the like and combinations thereof. Insoluble lubricants can include but are not limited to magnesium stearate, stearic acid, glyceryl palmitostearate, sodium stearyl fumarate and the like and combinations thereof. The amount of the lubricant depends on the lubricating effects desired and is well known to those of ordinary skill in the art. For example, the amount of the lubricant in the composition can be from 0.1% to 0.5% w/w, preferably from 0.1% to 0.3% w/w of the composition. Suitable flavoring substances include but are not limited to natural flavors such as natural oils, extracts from plants, leaves, flowers, fruits and so forth and artificial flavors such as synthetic flavor oils and flavoring aromatics and so forth and combinations thereof. These may include cinnamon oil, oil of wintergreen, peppermint oils, clove oil, bay oil, anise oil, eucalyptus, thyme oil, cedar leaf oil, oil of nutmeg, oil of sage, oil of bitter almonds, cassia oil and the like. Also useful as flavors are vanilla, citrus oil, including lemon, orange, grape, lime and grapefruit, and fruit essences, including apple, pear, peach, strawberry, raspberry, cherry, plum, pineapple, apricot and so forth. Flavors which have been found to be particularly useful include but are not limited to commercially available orange, grape, cherry and bubble gum flavors and mixtures thereof. The amount of flavoring may depend on a number of factors, including the organoleptic effect desired. The amount of the flavoring substance in the composition can be from about 0.01% to 0.2% w/w, preferably from 0.01% to 0.1% w/w of the composition.

Useful coloring agents include food, drug and cosmetic (FD&C) colors, for example- dyes, pigments, lakes, natural colorants and derived colorants. Useful lakes include dyes adsorbed on aluminum hydroxide and other suitable carriers. Examples of suitable colors include FD&C Red No. 3, FD&C Red No. 40, FD&C Blue No. 1, FD&C Blue No. 2, FD&C Yellow No. 5, FD&C Yellow No. 6, FD&C Green No. 3 and combinations thereof. The amount of coloring agent depends on the aesthetic appearance desired and is well known to those skilled in the art.

In accordance with an embodiment, the present disclosure relates to a RDT composition comprising Sitagliptin phosphate. In a further embodiment, the Sitagliptin phosphate is anhydrous.

In accordance with an embodiment, the amount of Sitagliptin in the RDT composition can be in the range of 1 - 100 mg. More preferably, the amount of Sitagliptin is the therapeutically effective amount.

In accordance with an embodiment, the amount of Sitagliptin in said RDT composition is in the range of 25 - 100 mg.

In accordance with an embodiment, the present disclosure relates to a rapidly disintegrating tablet composition comprising Sitagliptin in an amount of about 25 - 100 mg and an acid-base couple wherein, sodium content of the composition is in the range of 0.1- 10 mg and potassium content is less than 15 mg. More preferably, sodium content of the said composition is in the range of 1- 10 mg and potassium content is less than 15 mg.

In accordance with a further embodiment, the present disclosure relates to an RDT composition comprising Sitagliptin in an amount of about 25 -100 mg and an acid-base couple, wherein the composition has a sodium content less than 10 mg and a potassium content less than 15 mg and wherein, the RDT composition is prepared using a twin screw processor.

In accordance with an embodiment, the present disclosure relates to a RDT composition comprising Saxagliptin hydrochloride. In a further embodiment, Saxagliptin hydrochloride is a monohydrate.

In accordance with an embodiment, the amount of Saxagliptin in the RDT composition is in the range of 1 - 50 mg.

In accordance with another embodiment, the amount of Saxagliptin in the RDT composition is in the range of 2-5 mg.

In accordance with an embodiment, the present disclosure relates to a rapidly disintegrating tablet composition comprising Saxagliptin in an amount of about 2 - 5 mg and an acid-base couple wherein, sodium content of the composition is in the range of 0.1-10 mg and potassium content is less than 15 mg. More preferably, sodium content of the said composition is in the range of 1- 10 mg and potassium content is less than 15 mg.

In accordance with a further embodiment, the present disclosure relates to an RDT composition comprising Saxagliptin in an amount of about 2 - 5 mg and an acid-base couple, wherein the composition has a sodium content less than 10 mg and a potassium content less than 15 mg and wherein, the RDT composition is prepared using a twin screw processor.

In accordance with an embodiment, the RDT compositions of the present disclosure exhibit a disintegration time of less than 60 seconds, preferably less that 40 seconds in water at room temperature of about 25 °C ± 5 °C. In accordance with an exemplary embodiment, the RDT compositions have a disintegration time of 15 to 30 seconds in water at room temperature of about 25 °C ± 5 °C. The RDT compositions of the present disclosure exhibit a disintegration time of less than 60 seconds, preferably less than 40 seconds in less than 10 ml of water at room temperature of about 25 °C ± 5 °C. The RDT compositions of the present disclosure may also exhibit a disintegration time of less than 60 seconds, preferably less than 40 seconds in less than 6 ml of water at room temperature of about 25 °C ± 5 °C. After disintegration, the compositions of present disclosure may be in the form of a solution or a dispersion. The solubility of API (DPPIV inhibitor) as well as the co-formulated excipients in water, determines the formation of a clear or hazy solution or a uniform dispersion.

In accordance with an embodiment, the RDT composition of the present disclosure has a hardness of at least 2 kp (kilopond). In accordance with an exemplary embodiment, the tablet has a hardness of 2 to 20 kp.

In accordance with an embodiment, the RDT composition of the present disclosure exhibit a disintegration time not more than 120 seconds for upto 50% loss of porosity.

In accordance with an embodiment, the RDT composition of the present disclosure exhibit a disintegration time not more than 120 seconds for loss of porosity between 30-50%. In accordance with an embodiment, the RDT composition of the present disclosure exhibit a disintegration time not more than 60 seconds for loss of porosity between 30-45 %.

Any conventional compression techniques well known to the person skilled in the art may be used to form the RDT compositions of the present disclosure.

The invention of the present disclosure is exemplified by the following nonlimiting examples.

## EXAMPLES

RDT compositions of DPP-IV inhibitors were prepared as per examples mentioned below. Retained $CO_2$ content, bulk and tap density and compressibility of the input blend and granules and disintegration time of the tablets were determined by the methods given below:

### Analytical methods:

Determination of Retained $CO_2$ Content: 5.0 g of input blend and 5.0 g of granules were added in two separate 100 ml 10% v/v sulfuric acid solutions. The loss in weight of the input blend and granules due to liberation of CO2 as a result of the reaction between sulfuric acid and bicarbonate or carbonate present in the blend or granules was recorded. The difference between the weight of the input blend and granules after reaction and the initial weight of input blend and granules respectively was also calculated to measure the retained CO2 content (g) in the granules. The relative percentage of retained CO2 content in the granules was then calculated using the formula below:

$$\text{Relative retained } CO_2 \text{ content (\%)} = (\text{Observed Weight (g) of } CO_2 \text{ in granules X } 100) / \text{Observed Weight (g) of } CO_2 \text{ in input blend}$$

Measurement of Bulk and Tap Density: A known amount (m) of the input blend and the granules were added into two separate dry graduated 100 ml cylinders. The contents of the two cylinders were leveled without compacting; and the unsettled apparent bulk volume ($V_0$) to the nearest graduated unit was read.
The bulk density (g/ml) was calculated using the formula:

$$\text{Bulk Density} = \text{Mass (m) / Bulk Volume } (V_0)$$

To calculate the tap density, the contents of the two cylinders were tapped mechanically up to a maximum of 1250 taps. The tap density was calculated using the formula:

$$\text{Tap Density} = \text{Mass (m) / Tapped Volume } (V_t)$$

Measurement of Compressibility: Compressibility was measured in terms of Hausner ratio and Compressibility Index, as provided in Hausner, H.H., Int. J. Powd. Metall., Vol 3, pg 7, 1967 and Carr, R.L., Chem. Eng., Vol 72, Issue 1, pg 163 and Issue 2, pg 69, 1965, respectively.

Determination of disintegration time:

Tablets were dropped in 10 mL beaker containing 6 mL of purified water at 20 °C $\pm$ 5 °C and the time taken for the tablets to disperse/disintegrate was recorded.

Determination of % loss in Porosity:

There is a loss in porosity as the blend comprising essentially of API (DPPIV inhibitor) and acid-base couple is compressed into tablets.
The % loss in porosity of the blend (ready for compression into tablets) was determined by using the following formula:

$$\% \text{ loss in Porosity} = ((1/\text{density of compact} - 1/\text{density of tablet}) \text{ X } 100)) \div (1/ \text{ density of compact})$$

$$\text{Compact density} = \text{Tapped density of the blend before tableting}$$

**Composition of granules containing citric acid anhydrous, sodium bicarbonate and sodium carbonate**

Table 1

| Ingredients | % w/w |
|---|---|
| Citric acid anhydrous (CAA) | 44.5 |
| Sodium bicarbonate (SBC) | 50.3 |
| Sodium carbonate (SC) | 5.2 |
| **Total** | **100.00** |

Citric acid anhydrous, sodium bicarbonate and sodium carbonate were dried in an oven at 45 ° C for 3 hours. The dried ingredients were weighed as per the formula and passed separately through ASTM #60 mesh. The sifted ingredients were blended together manually in a polybag and immediately processed in a co-rotating twin screw processor using the following processing conditions.

**Machine:** Omega 20.
**L/D:** 30
**Processing area relative humidity:** 27-28%;
**Processing area temperature:** 28°C
**Screw Speed:** 600 rpm.
**Feed rate:** 10 Hz (18 kg/hr).
**Observed Torque:** 9 -13%

**Screw Configuration:**

Table 2

| Elemen ts | RSE 15/15 | NRF 40/20 | RFV-40/40 | RFN-40/20 | RSE-30/15 | RSE-15/30 | RSE-15/15 | RSE-20/20 |
|---|---|---|---|---|---|---|---|---|
| **No's** | 1 | 1 | 4 | 1 | 1 | 8 | 7 | 1 |

**Barrel Temperature (°C)**

Table 3

| B1 | B2 | B3 | B4 | B5 |
|---|---|---|---|---|
| 30 | 30 | 140 | 140 | 140 |

Table 4 gives the comparison data of physical parameters for the input blend and the granules.

Table 4

| Parameter | Input Blend | Granules |
|---|---|---|
| LOD (%w/w) (75°C for 5 minutes) | 0.13 | --- |
| Observed Weight (g) of $CO_2$ | 1.62 | 1.51 |
| Relative Carbon dioxide content (%)[#] | ---- | 93.21 |
| Bulk density (g/cc) | 0.806 | 0.558 |
| Tapped density (g/cc) | 1.186 | 0.731 |

(continued)

| Parameter | Input Blend | Granules |
|---|---|---|
| Compressibility Index (%) | 32.075 | 23.636 |
| Hausner's ratio | 1.472 | 1.31 |
| Particle Size Distribution (Median Diameter in microns) | --- | 360 |

# % Relative retained $CO_2$ content for placebo granules containing citric acid = Observed Weight (g) of $CO_2$ in granules/ Observed Weight (g) of $CO_2$ in input blend X 100 = 1.51/1.62 $\times$ 100 = 93.21%

**Composition of granules containing citric acid anhydrous and potassium bicarbonate**

Table 5

| Ingredients | % w/w |
|---|---|
| Citric acid anhydrous (CAA) | 38.99 |
| Potassium bicarbonate (KBC) | 61.00 |
| **Total** | **100.00** |

**Procedure:**

Citric acid anhydrous was passed through #60 mesh and dried at 45 °C for 3 hours in a hot air oven. Potassium bicarbonate was dried in an oven at 45 °C for 3 hours. The dried ingredients were weighed as per the formula. Then the ingredients were blended together manually in a polybag and immediately processed in a co-rotating twin screw processor using the following processing conditions.

**Machine:** Omega 20.
**L/D:** 30
**Processing area relative humidity:** 22%; **Processing area temperature:** 29°C
**Screw Speed:** 600 rpm.
**Feed rate:** 10 Hz (19 kg/hr).
**Observed Torque:** 15 - 18%
**Screw Configuration:**

Table 6

| Elemen ts | RSE 15/15 | NRF 40/20 | RFV-40/40 | RFN-40/20 | RSE-30/15 | RSE-15/30 | RSE-15/15 | RSE-20/20 |
|---|---|---|---|---|---|---|---|---|
| **No's** | 1 | 1 | 4 | 1 | 1 | 8 | 7 | 1 |

**Barrel Temperature (° C) (Vent port open at Barrel "B4")**

Table 7

| B1 | B2 | B3 | B4 | B5 |
|---|---|---|---|---|
| 30 | 30 | 130 | 130 | 130 |

Table 8 gives the comparison data of physical parameters for the input blend and the granules.

Table 8

| Parameter | Input Blend | Granules |
|---|---|---|
| LOD (%w/w) (75° C for 5 mins) | 0.21 | --- |
| Observed Weight (g) of $CO_2$ | 1.49 | 1.24 |
| Relative Carbon dioxide content (%)$^\$$ | ---- | 83.22 |
| Bulk density (g/cc) | 0.89 | 0.593 |
| Tapped density (g/cc) | 1.37 | 0.709 |
| Compressibility Index (%) | 35.08 | 16.36 |
| Hausner's ratio | 1.54 | 1.196 |
| Particle Size Distribution (Median Diameter in microns) | 390 | 700 |
| $^\$$ % Relative retained $CO_2$ content for placebo granules containing citric acid =Observed Weight (g) of CO2 in granules/ Observed Weight (g) of CO2 in input blend X 100 = 1.24/1.49 X 100 = 83.22% | | |

**Common formulation process for examples 1-9:**

*Procedure for blend preparation:*

All ingredients specified in each example were passed through ASTM #60 mesh and mixed *manually* in a polybag. The ingredients *in italics* as mentioned in the examples are meant to be formulated separately as granules as per procedures mentioned herein, before passing through ASTM #60 mesh and mixing with the other ingredients. *Compression:* Tableting of the blend was performed on 12 station Riddhi Pharma rotary compression machine (model: RD3SH-12).

**Examples 1-4**

**Saxagliptin tablet compositions**

Table 9

| Ingredients | Example 1 | Example 2 | Example 3 | Example 4 (comparative) |
|---|---|---|---|---|
| | mg/Tab | mg/Tab | mg/Tab | mg/Tab |
| Saxagliptin hydrochloride monohydrate | 3.1 eq. to 2.5 mg Saxagliptin | 6.14 eq. to 5 mg Saxagliptin | 6.14 eq. to 5 mg Saxagliptin | 6.14 eq. to 5 mg Saxagliptin |
| *Citric acid anhydrous* | *20.1* | *20.05* | *37.6* | - |
| *Sodium bicarbonate* | *22.6 eq. to 6.19 mg sodium* | *22.6 eq. to 6.19 mg sodium* | *22.6 eq. to 6.19 mg sodium* | - |
| *Sodium carbonate* | *2.3 eq to 1.01 mg of sodium* | *2.32 eq to 1.01 mg of sodium* | *2.3 eq to 1.01 mg of sodium* | - |
| *Potassium bicarbonate* | - | - | *27.5 eq. to 10.72 mg potassium* | - |
| Pearlitol® flash | 100.7 | 97.65 | 52.7 | 142.7 |
| Strawberry flavor | 0.08 | 0.08 | 0.08 | 0.08 |
| Sucralose | 0.8 | 0.75 | 0.8 | 0.8 |
| Sodium stearylfumarate | 0.4 | 0.4 | 0.4 | 0.4 |

(continued)

| Ingredients | Example 1 | Example 2 | Example 3 | Example 4 (comparative) |
|---|---|---|---|---|
| | mg/Tab | mg/Tab | mg/Tab | mg/Tab |
| Total | 150.0 | 150.0 | 150.0 | 150.0 |

**Physical parameters of tablet after compression using 6.5 mm punches (FFBE punch) for Examples 1-4:**

Table 10

| Tablet Parameters | Example 1 | Example2 | Example 3 | Example 4 (comparative) |
|---|---|---|---|---|
| Tooling (mm, type) | 6.5, FFBE* | 6.5, FFBE | 6.5, FFBE | 6.5, FFBE |
| Target weight (mg) | 150 | 150 | 150 | 150 |
| Thickness (mm, n=5) | 3.61- 3.66 | 3.39-3.42 | 3.02 -3.05 | 3.85-3.93 |
| Hardness (kp, n=5) | 2.1-2.9 | 3.1-3.5 | 2.3-3.9 | 2.2-4.6 |
| Disintegration time (seconds, n=5) | 17-19 | 16-26 | 21-27 | 47-52** |
| *Flat faced bevel edge<br>**Disintegration time study for the tablets without acid-base couple required manual intermittent shaking to observe complete disintegration of the tablets. | | | | |

**Observation:**

Tablets without acid-base couple (Example 4) disintegrate but do not result into dispersion in the given volume of water. Additionally, manual stirring/mixing is required to disperse the tablet residue. The disintegration time for the tablets without acid-base couple is higher than the tablets with acid-base couple as per Examples 1-3. Also, the tablets with acid-base couple, form dispersion simultaneously after disintegration, without any tablet residue.

**Examples 5-9**

**Sitagliptin tablet compositions:**

Table 11

| Ingredients | Example 5 | Example 6 | Example 7 | Example 8 | Example 9 (comparative) |
|---|---|---|---|---|---|
| | mg/tablet | mg/tablet | mg/tablet | mg/tablet | mg/tablet |
| Sitagliptin phosphate anhydrous | 31.3 eq. to 25 mg sitagliptin | 62.5 eq. to 50 mg sitagliptin | 125.1 eq. to 100 mg Sitagliptin | 125.1 eq. to 100 mg Sitagliptin | 125.1 eq. to 100 mg Sitagliptin |
| *Citric acid anhydrous* | *10.0* | *10.0* | *20.1* | *37.6* | *-* |
| *Sodium bicarbonate* | *11.3 eq. to 3.09 mg of sodium* | *11.3 eq. to 3.09 mg of sodium* | *22.6 eq. to 6.19 mg of sodium* | *22.6 eq. to 6.19 mg of sodium* | *-* |
| *Sodium carbonate* | *1.2 eq. to 0.52 mg of sodium* | *1.2 eq. to 0.52 mg of sodium* | *2.3 eq. to 1.01 mg of sodium* | *2.3 eq. to 1.01 mg of sodium* | *-* |
| *Potassium bicarbonate* | *-* | *-* | *-* | *27.5 eq. to 10.72 mg potassium* | *-* |

(continued)

| Ingredients | Example 5 | Example 6 | Example 7 | Example 8 | Example 9 (comparative) |
|---|---|---|---|---|---|
| | mg/tablet | mg/tablet | mg/tablet | mg/tablet | mg/tablet |
| Pearlitol® flash | 95.0 | 63.8 | 127.5 | 82.5 | 172.5 |
| Strawberry flavor | 0.08 | 0.08 | 0.2 | 0.2 | 0.2 |
| Sucralose | 0.8 | 0.8 | 1.5 | 1.50 | 1.5 |
| Sodium stearyl fumarate | 0.4 | 0.4 | 0.8 | 0.8 | 0.8 |
| **Total** | 150 | 150 | 300 | 300 | 300 |

**Physical parameters of tablet after compression using 10 mm punches (FFBE punch) for Examples 5-9:**

Table 12

| Tablet Parameters | Example 5 | Example 6 | Example 7 | Example 8 | Example 9 (comparative) |
|---|---|---|---|---|---|
| **Tooling (mm, type)** | 6.5, FFBE | 6.5, FFBE | 10, FFBE | 10, FFBE | 10, FFBE |
| **Target weight (mg)** | 150 | 150 | 300 | 300 | 300 |
| **Thickness (mm, n=5)** | 3.59- 3.62 | 3.50-3.53 | 3.9 -3.97 | 3.62-3.88 | 4.09-4.2 |
| **Hardness (kp, n=5)** | 2.1-3.3 | 2.1-3.3 | 2.8-3.1 | 2.1-3.8 | 2.0-2.9 |
| **Disintegration time (seconds, n=5)** | 15-17 | 22-23 | 21-22 | 24-30 | 24-26* |
| **#Dispersion time (seconds, n=5)** | 15-17 | 22-23 | 21-22 | 24-30 | Not even after 5 minutes |
| * Disintegration time study for the tablets without acid-base couple required manual intermittent shaking to observe complete disintegration of the tablets. #Dispersion time = time taken to form uniform dispersion after disintegration | | | | | |

**Observation:**

Tablets without acid-base couple disintegrate but do not result into dispersion in the given volume of water, additionally manual stirring/mixing is required to disperse the tablet residue. The disintegration time for the tablets without acid-base couple is almost similar to the tablets with acid-base couple as per examples 5-8, but the latter tablets form dispersion simultaneously after disintegration without any residue of the tablet.

**Example 10**

**Preparation of Saxagliptin granules containing Sodium bicarbonate and Sodium carbonate as $CO_2$ source:**

Table 13

| Ingredients | % w/w |
|---|---|
| Saxagliptin hydrochloride monohydrate eq. to 9.77 gms of saxagliptin) | 12.01 |
| Citric acid anhydrous (CAA) | 39.2 |

(continued)

| Ingredients | % w/w |
|---|---|
| Sodium bicarbonate (SBC) | 44.25 |
| Sodium carbonate (SC) | 4.54 |
| **Total** | **100** |

**Procedure:**

Citric acid anhydrous, sodium bicarbonate and sodium carbonate were dried in an oven at 45 ° C for 3 hours. The dried ingredients and saxagliptin were weighed as per the formula and passed separately through ASTM #60 mesh. The sifted ingredients were blended together manually in a polybag and immediately processed in a co-rotating twin screw processor using the following processing conditions.

**Machine:** Omega 20.
**L/D:** 30
Processing area relative humidity: 25%;
**Processing area temperature: 29° C**
**Screw Speed:** 600.
**Feed rate:** 10 Hz (15 kg/hr).
**Observed Torque:** 4 -8%

**Screw Configuration:**

Table 14

| Elements | RSE 15/15 | NRF 40/20 | RFV 40/40 | RFN 40/20 | RSE 30/15 | RSE 15/30 | RSE 15/15 | RSE 20/20 |
|---|---|---|---|---|---|---|---|---|
| No's | 1 | 1 | 4 | 1 | 1 | 8 | 7 | 1 |

**Barrel Temperature (° C).** (Feeding done through Barrel "B1")

Table 15

| B1 | B2 | B3 | B4 | B5 |
|---|---|---|---|---|
| 30 | 30 | 130 | 130 | 130 |

**Comparative physical parameters for input blend and granules:**

Table 16

| Parameters | Input Blend | Granules |
|---|---|---|
| LOD (%w/w) (75°C for 5 mins) | 0.29 | 1.16 |
| Observed weight (g) of $CO_2$ | 1.12 | 0.9 |
| Relative carbon dioxide content (%) | - | 80.36 |
| Bulk density (g/cc) | 0.55 | 0.48 |
| Tapped density (g/cc) | 1.03 | 0.67 |
| Compressibility Index (%) | 45.83 | 28.57 |

(continued)

| Parameters | Input Blend | Granules |
|---|---|---|
| Hausner's ratio | 1.84 | 1.4 |
| Cumulative particles retained on ASTM #60 screen | - | 29.68% |

**Composition of the tablet:**

Table 17

| Ingredients | mg/Tab |
|---|---|
| *Saxagliptin hydrochloride monohydrate* | *6.1 eq. to 5 mg of Saxagliptin* |
| *Citric acid anhydrous* | *20.0* |
| *Sodium bicarbonate* | *22.6 eq. to 6.19 mg of sodium* |
| *Sodium carbonate* | *2.3 equivalent to 1.01 mg of sodium* |
| Pearlitol® flash | 97.7 |
| Strawberry flavor | 0.08 |
| Sucralose | 0.8 |
| Sodium stearyl fumarate | 0.4 |
| **Total** | 150 |

**Procedure for blend preparation:**

All ingredients specified in example were passed through ASTM #60 mesh and mixed manually in a polybag.

Table 18

| Tablet Parameters | Example 10 |
|---|---|
| **Tooling (mm, type)** | 6.5, FFBE |
| **Target weight (mg)** | 150 |
| **Thickness (mm, n=5)** | 3.72- 3.86 |
| **Hardness (kp, n=5)** | 2.0-2.9 |
| **Disintegration time (seconds, n=5)** | 19-21 |

**Observation:**

Tablets formulated with intragranular saxagliptin in granules using twin screw processor disintegrate and disperse within 30 seconds. Hence, there is no change in disintegration of the tablet formulated with intra or extra granular saxagliptin.

**Example 11- Trials for estimating the effect of porosity on disintegration time Saxagliptin Tablet Composition**

Table 19

| Ingredients | mg/Tablet |
|---|---|
| Saxagliptin hydrochloride monohydrate | 6.14 eq. to 5 mg Saxagliptin |

(continued)

| Ingredients | mg/Tablet |
|---|---|
| *Citric acid anhydrous* | *20.05* |
| *Sodium bicarbonate* | *22.6 eq. to 6.19 mg sodium* |
| *Sodium carbonate* | *2.32 eq to 1.01 mg of sodium* |
| Pearlitol® flash | 97.65 |
| Flavor | 0.08 |
| Sucralose | 0.75 |
| Sodium stearyl fumarate | 0.4 |
| **Total** | 150.0 mg |

Compact density (Tapped density of the blend before Tabletting): 0.797 g/cc.

**Procedure for blend preparation:**

All ingredients specified in each example were passed through ASTM #60 mesh and mixed. The ingredients *in italics* as mentioned in the examples are meant to be formulated separately as granules as per procedures mentioned herein, before passing through ASTM #60 mesh and mixing with the other ingredients. **Compression:** Tableting of the blend was performed on 12 station Riddhi Pharma rotary compression machine (model: RD3SH-12) with tablet weight of 150 mg using 6.5 mm Flat faced bevel edge (FFBE).

**Physical parameters of tablet after compression using 6.5 mm punches (FFBE punch) at various hardness levels:**

Table 20

| Tablet Parameters | Example-11A | Example-11B | Example- 2 | Example-11C | Example-11D |
|---|---|---|---|---|---|
| Avg wt (mg) | 151.2 | 154.2 | 148.2 | 146.2 | 148.8 |
| Thickness (mm, n=5) | 3.03 -3.09 (Avg: 3.07) | 3.15 - 3.22 (Avg: 3.18) | 3.39 - 3.42 (Avg:3.40) | 3.41-3.51 (Avg: 3.47) | 3.76 - 3.81 (Avg: 3.79) |
| Hardness (kp, n=5) | 9.5 - 14.4 (Avg: 11.7) | 6.9 - 9.9 (Avg: 8.5) | 3.1-3.5 (Avg: 3.4) | 1.4-2.8 (Avg: 2.1) | 1.1 -1.8 (Avg: 1.3) |
| Disintegration time (seconds, n=3) | 80 - 86 (Avg: 83) | 58-60 (Avg: 59) | 26 - 16 (Avg: 20) | 15-20 (Avg: 18) | 17 -18 (Avg: 17) |
| Calculated Volume$ (mm³) | 101.81 | 105.65 | 112.95 | 115.14 | 125.63 |
| Calculated Density# (g/cc) | 1.485 | 1.460 | 1.312 | 1.270 | 1.184 |
| % Loss of porosity (when blend is compacted into a tablet)** | **46.4** | **45.6** | **39.2** | **36.8** | **32.8** |

$ Calculation for Volume of the Tablet = $\pi r^2 h$ (where r is the radius of tablets, h is the average thickness of the tablet)
# Calculation for Density: Average weight of tablet /calculated volume of the tablet
**% Loss of porosity when the blend is compacted into a tablet was calculated based on the calculated tablet density and density of compact (i.e. Tapped density of the blend ready for tableting)
% loss in Porosity = (1/density of compact - 1/density of tablet) × 100) ÷ 1/ density of compact

**Calculation for percentage loss of porosity**

Example 11 A:

$$= ((1/ 0.797 - 1/1.485) \, x100)) \, / \, 1/0.797$$

$$= ((1.25 - 0.67) \, x100)) \, / \, 1.25$$

$$= 46.4\%$$

## SPECIFIC EMBODIMENTS ARE DESCRIBED BELOW:

A rapidly disintegrating tablet comprising a therapeutically effective amount of a DPP-IV inhibitor, and an acid-base couple, wherein the tablet has a sodium content less than 10 mg and a potassium content less than 15 mg.

Such tablet(s), wherein the DPP-IV inhibitors is selected from a group consisting of Sitagliptin, Saxagliptin, Linagliptin, Alogliptin, Vildagliptin, Teneligliptin, Gemigliptin, their pharmaceutically acceptable salts, and combinations thereof.

Such tablet(s), wherein the acid is selected from a group consisting of citric acid, tartaric acid, fumaric acid, malic acid, adipic acid, succinic acid, lactic acid, glycolic acid, alpha hydroxy acid, ascorbic acid, amino acid and their salts, and combinations thereof.

Such tablet(s), wherein the base is selected from a group consisting of carbonates, sesquicarbonates, bicarbonate salts of alkali metals or amino acids, and combinations thereof.

Such tablet(s), wherein the base is selected from a group consisting of potassium carbonates, sodium carbonate, calcium carbonate, potassium bicarbonate, sodium bicarbonate, calcium bicarbonate, L-lysine carbonate, arginine carbonate, sodium glycine carbonate, and combinations thereof.

Such tablet(s), wherein the acid and the base are present in a molar ratio in the range of 1:6 to 3:1.

Such tablet(s), wherein the acid and the base are present in a molar ratio in the range of 1:2.5 to 1:3.5.

Such tablet(s), wherein the composition has a disintegration time of less than 60 seconds in 10 ml of water at a temperature of about 25 °C $\pm$ 5 °C.

## FURTHER SPECIFIC EMBODIMENTS ARE DESCRIBED BELOW:

A process for preparation of a rapidly disintegrating tablet comprising a DPP-IV inhibitor, the process comprising feeding an input blend comprising an acid-base couple, and optionally a DPP-IV inhibitor, into a co-rotating twin screw processor, melting only a portion of the acid to serve as an *in situ* granulating agent, granulating the input blend to form granules, collecting granules from the co-rotating twin screw processor, mixing the granules with one or more excipients and optionally, with a DPP-IV inhibitor to obtain a blend, and compressing the blend to form one or more tablets, wherein the DPP-IV inhibitor is either added to the input blend or mixed with the granules.

## FURTHER SPECIFIC EMBODIMENTS ARE DESCRIBED BELOW:

A rapidly disintegrating tablet comprising a therapeutically effective amount of a DPP-IV inhibitor, and an acid-base couple, wherein the disintegration time of the tablet does not exceed 120 seconds for upto 50% loss of porosity.

## FURTHER SPECIFIC EMBODIMENTS ARE DESCRIBED BELOW:

A rapidly disintegrating tablet comprising a therapeutically effective amount of a DPP-IV inhibitor, and an acid-base couple, wherein the disintegration time of the tablet does not exceed 120 seconds for loss of porosity between 30-50%.

Such tablet(s) wherein the disintegration time of the tablet does not exceed 60 seconds for loss of porosity between 30-45 %.

## INDUSTRIAL APPLICABILITY

The present disclosure provides rapidly disintegrating tablet compositions of DPP-IV inhibitors comprising acid-base couple and having mineral content below the daily intake limits with rapid disintegration and adequate mechanical strength. The content of minerals like sodium and potassium in the compositions is significantly below the daily intake limits recommended in case of Type 2 diabetes patients. The present disclosure also provides a simple and convenient continuous process for preparing rapidly disintegrating tablet compositions which can be conveniently administered by oral disintegration or as a dispersion or solution.

**Claims**

1.  A rapidly disintegrating tablet comprising:

    a therapeutically effective amount of a DPP-IV inhibitor, and
    an acid-base couple,

    wherein the tablet has a sodium content in the range of 1 mg to less than 10 mg and a potassium content less than 15 mg.

2.  The tablet as claimed in claim 1, wherein the DPP-IV inhibitors is selected from a group consisting of Sitagliptin, Saxagliptin, Linagliptin, Alogliptin, Vildagliptin, Teneligliptin, Gemigliptin, their pharmaceutically acceptable salts, and combinations thereof.

3.  The tablet as claimed in claim 1, wherein the acid is selected from a group consisting of citric acid, tartaric acid, fumaric acid, malic acid, adipic acid, succinic acid, lactic acid, glycolic acid, alpha hydroxy acid, ascorbic acid, amino acid and their salts, and combinations thereof.

4.  The tablet as claimed in claim 1, wherein the base is selected from a group consisting of carbonates, sesquicarbonates, bicarbonate salts of alkali metals or amino acids, and combinations thereof.

5.  The tablet as claimed in claim 1, wherein the base is selected from a group consisting of potassium carbonates, sodium carbonate, calcium carbonate, potassium bicarbonate, sodium bicarbonate, calcium bicarbonate, L-lysine carbonate, arginine carbonate, sodium glycine carbonate, and combinations thereof.

6.  The tablet as claimed in claim 1, wherein the acid and the base are present in a molar ratio in the range of 1:6 to 3: 1.

7.  The tablet as claimed in claim 6, wherein the acid and the base are present in a molar ratio in the range of 1:2.5 to 1:3.5.

8.  The tablet as claimed in claim 1, wherein the composition has a disintegration time of less than 60 seconds in 10 ml of water at a temperature of about 25 °C $\pm$ 5 °C.

9.  A process for preparation of a rapidly disintegrating tablet comprising a DPP-IV inhibitor as claimed in claim 1, the process comprising:

    feeding an input blend comprising an acid-base couple, and optionally a DPP-IV inhibitor, into a co-rotating twin screw processor;
    melting only a portion of the acid to serve as an in situ granulating agent; granulating the input blend to form granules;
    collecting granules from the co-rotating twin screw processor;
    mixing the granules with one or more excipients and optionally, with a DPP-IV inhibitor to obtain a blend; and
    compressing the blend to form one or more tablets,
    wherein the DPP-IV inhibitor is either added to the input blend or mixed with the granules.

10. The tablet as claimed in claim 1 comprising:

    a therapeutically effective amount of a DPP-IV inhibitor, and
    an acid-base couple,

    wherein the disintegration time of the tablet does not exceed 120 seconds for upto 50% loss of porosity.

11. The tablet as claimed in claim 1 comprising:

    a therapeutically effective amount of a DPP-IV inhibitor, and
    an acid-base couple,

    wherein the disintegration time of the tablet does not exceed 120 seconds for loss of porosity between 30-50%.

**12.** The tablet as claimed in claim 10 or 11, wherein the disintegration time of the tablet does not exceed 60 seconds for loss of porosity between 30-45 %.

**Patentansprüche**

**1.** Schnell zerfallende Tablette, umfassend:

eine therapeutisch wirksame Menge eines DPP-IV-Hemmers, und
ein Säure-Base-Paar,

wobei die Tablette einen Natriumgehalt im Bereich von 1 mg bis kleiner als 10 mg und einen Kaliumgehalt kleiner als 15 mg aufweist.

**2.** Tablette nach Anspruch 1, wobei der DPP-IV-Hemmer ausgewählt ist aus einer Gruppe bestehend aus Sitagliptin, Saxagliptin, Linagliptin, Alogliptin, Vildagliptin, Teneligliptin, Gemigliptin, ihren pharmazeutisch akzeptablen Salzen, und Kombinationen davon.

**3.** Tablette nach Anspruch 1, wobei die Säure ausgewählt ist aus einer Gruppe bestehend aus Citronensäure, Weinsäure, Fumarsäure, Äpfelsäure, Adipinsäure, Bernsteinsäure, Milchsäure, Glykolsäure, $\alpha$-Hydroxysäure, Ascorbinsäure, Aminosäure und ihren Salzen, und Kombinationen davon.

**4.** Tablette nach Anspruch 1, wobei die Base ausgewählt ist aus einer Gruppe bestehend aus Carbonaten, Sesquicarbonaten, Bicarbonatsalzen von Alkalimetallen oder Aminosäuren, und Kombinationen davon.

**5.** Tablette nach Anspruch 1, wobei die Base ausgewählt ist aus einer Gruppe bestehend aus Kaliumcarbonat, Natriumcarbonat, Calciumcarbonat, Kaliumhydrogencarbonat, Natriumhydrogencarbonat, Calciumhydrogencarbonat, L-Lysincarbonat, Arginincarbonat, Natriumglycincarbonat, und Kombinationen davon.

**6.** Tablette nach Anspruch 1, wobei die Säure und die Base in einem Molverhältnis im Bereich von 1:6 bis 3:1 vorliegen.

**7.** Tablette nach Anspruch 6, wobei die Säure und die Base in einem Molverhältnis im Bereich von 1:2,5 bis 1:3,5 vorliegen.

**8.** Tablette nach Anspruch 1, wobei die Zusammensetzung eine Zerfallszeit von weniger als 60 Sekunden in 10 ml Wasser bei einer Temperatur von etwa 25 °C $\pm$ 5 °C aufweist.

**9.** Verfahren zur Herstellung einer schnell zerfallenden Tablette, umfassend einen DPP-IV-Hemmer, nach Anspruch 1, wobei das Verfahren umfasst:

Einfüllen eines Einsatzgemisches, umfassend ein Säure-Base-Paar und gegebenenfalls einen DPP-IV-Hemmer, in eine gleichläufige Doppelschneckenmaschine;
Schmelzen nur eines Teils der Säure, um als Mittel zur Granulierung *in situ* zu dienen; Granulieren des Einsatzgemisches, um Granulatkörner zu bilden;
Sammeln der Granulatkörner aus der gleichläufigen Doppelschneckenmaschine;
Mischen der Granulatkörner mit einem oder mehreren Exzipienten und gegebenenfalls mit einem DPP-IV-Hemmer, um ein Gemisch zu erhalten;
und
Zusammenpressen des Gemisches, um eine oder mehrere Tabletten zu bilden, wobei der DPP-IV-Hemmer entweder zu dem Einsatzgemisch hinzugefügt oder mit den Granulatkörnern gemischt wird.

**10.** Tablette nach Anspruch 1, umfassend:

eine therapeutisch wirksame Menge eines DPP-IV-Hemmers, und
ein Säure-Base-Paar,

wobei die Zerfallszeit der Tablette bei bis zu 50 % Porositätsverlust 120 Sekunden nicht übersteigt.

**11.** Tablette nach Anspruch 1, umfassend:

eine therapeutisch wirksame Menge eines DPP-IV-Hemmers, und
ein Säure-Base-Paar,

wobei die Zerfallszeit der Tablette bei einem Porositätsverlust zwischen 30-50 % 120 Sekunden nicht übersteigt.

**12.** Tablette nach Anspruch 10 oder 11, wobei die Zerfallszeit der Tablette bei einem Porositätsverlust zwischen 30-45 % 60 Sekunden nicht übersteigt.

**Revendications**

**1.** - Comprimé à délitement rapide comprenant :

une quantité thérapeutiquement efficace d'un inhibiteur de DPP-IV, et
un couple acide-base,

dans lequel le comprimé a une teneur en sodium se situant dans la plage allant de 1 mg à moins de 10 mg et une teneur en potassium inférieure à 15 mg.

**2.** - Comprimé selon la revendication 1, dans lequel les inhibiteurs de DPP-IV sont choisis dans un groupe consistant en la Sitagliptine, la Saxagliptine, la Linagliptine, l'Alogliptine, la Vildagliptine, la Ténéligliptine, la Gémigliptine, leurs sels pharmaceutiquement acceptables, et des combinaisons de ceux-ci.

**3.** - Comprimé selon la revendication 1, dans lequel l'acide est choisi dans un groupe consistant en l'acide citrique, l'acide tartrique, l'acide fumarique, l'acide malique, l'acide adipique, l'acide succinique, l'acide lactique, l'acide glycolique, l'acide alpha hydroxy, l'acide ascorbique, un acide aminé et leurs sels, et des combinaisons de ceux-ci.

**4.** - Comprimé selon la revendication 1, dans lequel la base est choisie dans un groupe consistant en des carbonates, des sesquicarbonates, des sels bicarbonate de métaux alcalins ou d'acides aminés, et des combinaisons de ceux-ci.

**5.** - Comprimé selon la revendication 1, dans lequel la base est choisie dans un groupe consistant en des carbonates de potassium, du carbonate de sodium, du carbonate de calcium, du bicarbonate de potassium, du bicarbonate de sodium, du bicarbonate de calcium, du carbonate de L-lysine, du carbonate d'arginine, du carbonate de sodium glycine, et des combinaisons de ceux-ci.

**6.** - Comprimé selon la revendication 1, dans lequel l'acide et la base sont présents dans un rapport molaire se situant dans la plage allant de 1:6 à 3:1.

**7.** - Comprimé selon la revendication 6, dans lequel l'acide et la base sont présents dans un rapport molaire se situant dans la plage allant de 1:2,5 à 1:3,5.

**8.** - Comprimé selon la revendication 1, dans lequel la composition a un temps de délitement inférieur à 60 secondes dans 10 ml d'eau à une température d'environ 25°C ± 5°C.

**9.** - Procédé de préparation d'un comprimé à délitement rapide comprenant un inhibiteur de DPP-IV selon la revendication 1, le procédé comprenant :

introduire un mélange d'entrée comprenant un couple acide-base, et facultativement un inhibiteur de DPP-IV, dans un processeur à double vis co-rotatives ;
faire fondre seulement une partie de l'acide pour servir d'agent de granulation in situ ; granuler le mélange d'entrée pour former des granulés ;
collecter les granulés du processeur à double vis co-rotatives ;
mélanger les granulés avec un ou plusieurs excipients et facultativement avec un inhibiteur de DPP-IV pour obtenir un mélange ; et
comprimer le mélange pour former un ou plusieurs comprimés,
dans lequel l'inhibiteur de DPP-IV est soit ajouté au mélange d'entrée soit mélangé avec les granulés.

**10.** - Comprimé selon la revendication 1 comprenant

une quantité thérapeutiquement efficace d'un inhibiteur de DPP-IV, et
un couple acide-base,

dans lequel le temps de délitement du comprimé ne dépasse pas 120 secondes pour une perte de porosité allant jusqu'à 50 %.

**11.** - Comprimé selon la revendication 1 comprenant

une quantité thérapeutiquement efficace d'un inhibiteur de DPP-IV, et
un couple acide-base,

dans lequel le temps de délitement du comprimé ne dépasse pas 120 secondes pour une perte de porosité comprise entre 30 et 50 %.

**12.** - Comprimé selon la revendication 10 ou 11, dans lequel le temps de délitement du comprimé ne dépasse pas 60 secondes pour une perte de porosité comprise entre 30 et 45 %.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015040460 A1
- WO 2015044880 A1
- EP 2848241 A1
- IN 4527CHENP2014